# EUROPEAN PATENT SPECIFICATION

(11) **EP 1 438 378 B1**
(45) Date of publication and mention of the grant of the patent: **26.10.2011**
(21) Application number: 01980890.6
(22) Date of filing: 18.10.2001
(51) Int. Cl.: C11C 3/10, C12P 7/64, A23D 9/00, A23D 9/02

(54) **CHOLESTEROL LOWERING STRUCTURED LIPIDS WITH OMEGA 6 PUFA**
CHOLESTERINSENKENDE STRUKTURIERTE LIPIDE MIT OMEGA 6 PUFA
LIPIDES STRUCTURES ABAISSANT LE TAUX DE CHOLESTEROL AVEC OMEGA 6 PUFA

(43) Date of publication of application: 21.07.2004
(73) Proprietor: Council of Scientific and Industrial Research, New Delhi 110 001 (IN)
(72) Inventor: RAO, Reena, 570 013 Karnataka (IN); SAMBAIAH, Kari, 570 013 Karnataka (IN); LOKESH, Belur, Ramaswamy, 570 013 Karnataka (IN)
(74) Representative: Hart-Davis, Jason
(86) International application number: PCT/IN2001/000182
(87) International publication number: WO 2003/033632

(56) References cited:
- WO-A-00/47055
- US-A- 5 962 712
- US-A- 6 034 130
- PATENT ABSTRACTS OF JAPAN vol. 016, no. 437 (C-0984), 11 September 1992 (1992-09-11) & JP 04 152861 A (SNOW BRAND MILK PROD CO LTD), 26 May 1992 (1992-05-26)
- ZEITOUN M A M: "PHYSICAL PROPERTIES OF INTERESTERIFIED FAT BLENDS" JOURNAL OF THE AMERICAN OIL CHEMISTS' SOCIETY, AMERICAN OIL CHEMISTS' SOCIETY. CHAMPAIGN, US, vol. 70, no. 5, 1 May 1993 (1993-05-01), pages 467-471, XP000372582 ISSN: 0003-021X

## Description

### TECHNICAL FIELD

The present invention relates to a process for production of cholesterol lowering structured lipids rich in omega 6 polyunsaturated fatty acids from coconut oil, the structured lipids obtainable by said process and the use of these cholesterol lowering structured lipids.

### BACKGROUND ART

Coconut oil is a kernel oil which is a natural source of MCFA (53% of C 8: 0-C 12: 0). Its lauric acid content is very high (48%). The lauric fats provide high nutritional value because either the portal or lymphatic systems can absorb them. They provide excellent nutrition for critically ill patients and do not cause any undue coronary difficulties despite their saturation. In fact, the lauric fats provide unexpected usefulness in protein catabolism, yielding positive nitrogen balance and enhanced protein formation. But coconut oil contains very low levels of polyunsaturated fatty acids (PUFA) (1.8% of linoleic acid). Long term feeding of coconut oil as the sole source of fat in the diet of experimental rats has shown EFA deficiency symptoms characterized by scaly dermatitis, excessive water loss through the skin, impaired growth and reproduction and poor wound healing. In addition to this, myristic and palmitic acids that contribute to around 33% of the total fatty acids of coconut oil have been shown to be hypercholesterolemic which is a risk factor for cardiovascular disease.

Medium chain fatty acids (MCFA) comprise fatty acids with 6 to 12 carbon chain length. MCFA offer numerous health benefits. They are easily absorbed, transported via the portal system and rapidly metabolized to yield quick energy and is not deposited in the body as fat. Medium chain triglycerides (MCT) have clinical applications in the treatment of fat malabsorption disorders, gall bladder disease, hyperlipidemia, obesity and deficiency of the carnitine system. But MCT alone cannot function as an ideal fat source for humans as they do not provide essential fatty acids (EFA) (EFA cannot be synthesized by the body and must be therefore ingested in the diet).

Linoleic acid is an essential omega 6 PUFA as it cannot be synthesized by mammals.

Linoleic acid can be found in seeds of most plants except coconut, cocoa and palm nuts. It is utilized for the synthesis of complex lipids that provide the permeability barrier to the epidermis. They also help to maintain optimum levels of unsaturation in tissue lipids. It has been found to have a reducing effect on plasma cholesterol and an inhibitory effect on arterial thrombus formation. In the body, linoleic acid is metabolized to form arachidonic acid, which is a substrate for eicosanoid biosynthesis. Structured lipids are triacylglycerols containing mixtures of short-, medium-, and long-chain fatty acids attached to a glycerol backbone for specific functionality. Structured lipids are formed by
(a) hydrolysis and esterification;
(b) interesterification;
(c) lipase-interesterification;
(d) traditional chemical methods or
(e) genetic manipulation.

The structured lipids are particularly useful because of the way in which they are metabolized. Specific fatty acids can be attached to specific portions of the glycerol backbone to ensure that these fatty acids are absorbed in a specific manner in the digestive process.

Enzymatic acidolysis with specific lipase provides an efficient method to improve the nutritional and physical properties of lipids by incorporating the required acyl groups into specific positions of the triacylglycerols.

A physical blend of MCFA rich triacylglycerols and PUFA rich triacylglycerols does not improve the absorption or metabolism of the fatty acids since each of the individual triacylglycerol maintains its original absorption rates.

Safflower oil is a natural source of linoleic acid (68% of total fatty acids). Under controlled reaction conditions employing immobilized sn-1-3 specific microbial lipases, the saturated fatty acid of coconut oil can be partially replaced to incorporate the required amount of omega 6 PUFA from free fatty acids of safflower oil thus obtaining a unique structured lipid rich in MCFA and omega 6 PUFA.

Because of faster absorption, Medium chain triglycerides (MCT) are useful as a calorie source in the treatment of hospitalized patients. Some hospitalized patients, particularly critically ill patients, require total parenteral nutrition and have a high risk of infection. These patients often have difficulty in obtaining the proper amount of nutrients and energy from the diet; a diet that both minimizes the risk of infection and provides quick nutrition would be of vast benefit to these patients. These diets must provide the essential fatty acids, including a limited amount of specific omega-6 fatty acids

It has been theorized that a structured lipid containing a medium chain fatty (C.6-C.12) acid residue may provide improved absorption of other fatty acids attached to the structured lipid. A recent paper by Jensen (Am. J. Clin. Nutr.1992, 60: 518-524) disclosed that a structured lipid containing medium chain fatty acid residues and long chain fatty acid residues (omega 3 fatty acids from fish oil) are absorbed faster by the body than the physical mixture of the same fatty acids. There is no suggestion or teaching that a specific structured lipid containing MCFA and omega 6 PUFA would be useful to modify the lipid profile of the body.

Reference may be made to an article, Kaimal, and Saroja, (1989, Journal of Oil Technologist's Association of India, Jan- Mar. 2-10). Wherein coconut oil was modified by enzymatic transesterification with safflower oil. The drawbacks of this method are that the products could not be expected to have specific type of fatty acid composition as two types of triacylglycerols were involved and the incorporation was poor as only preliminary studies were carried out.

Reference may also be made to the article by Shimada, Y., Sugihara, A., Maruyama, K., Nagao, T., Nakayama, S., Nakano H., and Tominaga, Y., (1996) J. Am. Oil Chem. Soc.73, 1415-1420, wherein safflower oil or linseed oil were subjected to enzymatic acidolysis to produce structured lipids containing both EFA and MCFA. But the draw back of this invention is that the MCFA was a highly purified one obtained commercially which is not economical.

Similarly, reference may also be made to the article by Akoh C.C.,.Jennings B.H and. Lillard. D.A, (1996) J. Am. Oil Chem. Soc. 72, 1059-1062 who used EPA ethyl esters (omega 3 PUFA) to modify evening primrose oil which is a rich source of γ-linolenic acid, an omega 6 PUFA. The drawbacks of this invention were that, the structured lipid developed in this case had very low levels of MCFA and serves only as a single rich source of both omega 3 and omega 6 PUFA rather than aiming at optimal nutrition. Moreover, the EPA ethyl esters used are from commercial sources.

Reference may be made to Lee, K-T and Akoh C.C., (J.Food Biochem. 1999, 23.197-208) wherein structured lipids were synthesized from synthetic tricaprylin and fish oil free fatty acids rich in omega 3 PUFA. The product when fed to mice for 21 days showed lower levels of serum total cholesterol, LDL cholesterol and triacylglycerol in comparison with soybean oil. However it is not very clear from this study whether a physical mix (blending) of tricaprylin and fish oil (having same fatty acid composition as the structured lipids) would have the same effect as the structured lipids on the serum lipid profile. The soybean oil used as a control in this study does not provide a good comparison with the structured lipid, which had a different fatty acid composition.

U.S. Pat. No. 5,661,180 to DeMichele, et al., describes a structured lipid, which provides substantial benefits in terms of modifying the prostanoid synthesis pathway, resulting in an improved response to endotoxic shock and other stress states. This structured lipid includes three components formed on a glycerol backbone. The first component is either alpha-linolenic acid or dihomogamma-linolenic acid. The second component is a medium chain (C 6 - C 12) fatty acid residue and the third component is a C 18 - C 22 fatty acid residue. The structured lipids in this case are prepared from a physical blend of oils subjected to a transesterification reaction to yield a reaction product that contains the structured lipids of this invention. The draw back of this is that the fatty acids will be randomized among the triglycerides of the two oils selected and there will not be any specificity in the positioning of fatty acids in the structured lipids.

Canadian Patent Application 2000391 with a WPI Accession Number of 90-139962/19 discloses the triglyceride 2-(alpha-linolenoyl)/gamma-linolenoyl)-1,3-di (octanoyol/decanoyl) glycerol as useful in nutritional lipids. It is suggested that these triglycerides are useful as components in nutritional compositions. The fatty acids are essential for control of tonus of smooth-muscle cells in the blood vessels or the tonus of the smooth muscle cells in the lungs and thus are useful in the control of respiratory distress. This reference neither suggests nor discloses the specific structured lipids of this invention nor the methods of using them.

European Patent Application Number 87114297.2 discloses a triglyceride having a C.8 to C. 14 fatty add residue at the 2-position of the triglyceride and a residue of C. 18 or higher fatty acids at the 1 and 3 positions thereof. There is no suggestion or disclosure of the specific structured lipids of the invention or the benefits that can be realized by feeding the structured lipids of this invention.

JP Pat. No. 04152861 discloses a composition containing mainly (A) a fat having a fatty acid composition and (B) a protein composed of a milk protein and soybean protein or decomposition product of the protein or an amino acid. The fat having a fatty acid composition is composed of medium chain fatty acid (e.g. caprylic acid), omega 3 and omega 6 fatty acids.

US Patent No. 5,962,712 deals with structured lipid containing gamma-linolenic or dihomogamma-linolenic fatty acid residue, a medium chain (C6-C12) fatty acid residue and n-3 fatty acid residue. This structured lipid is particularly well adapted to the treatment of diseases or stress states. These two last documents do not relate to fats rich in lauric acid.

U.S. Pat. No. 6,034,130 discloses a synthetic lipid composition containing less than 2% by weight of free fatty acids. This lipid composition is close to that of human milk and is synthesized under a process for incorporating PUFAs. In this last document, the content of omega 3 and omega 6 PUFA is relatively low.

### DISCLOSURE OF THE INVENTION

The main object of the present invention is to provide a process for the synthesis of unique structured lipids rich in omega 6 polyunsaturated fatty acids using natural sources.

Yet another object of the present invention is to provide a process for the synthesis of structured lipids that are rich in MCFA and omega 6 PUFA, which is nutritionally advantageaous by way of being hypocholesterolemic and hypotriglyceridemic.

Still, another object of the present invention is to provide a process for the synthesis of structured lipids rich in omega 6 polyunsaturated fatty acids that could be clinically administered to patients in parenteral nutrition.

Further another object of the present invention is to provide a process of Enzymatic acidolysis to produce fats (structured lipids) with a better triglyceride-distribution than known natural fats.

Yet, another object of the present invention is to provide a process for structured lipids with an improved melting behaviour as they will hardly contain any trisaturated triglycerides,

Yet another object of the present invention is to provide a process to develop a product, for use in a controlled diet for critically ill patients, comprising lauric acid to provide quick energy and n-6 PUFA to modulate their eicosanoid production especially in immune compromised patients and linoleic acid to take care of EFA requirement.

Yet another object of the present invention is to develop a product by modifying coconut oil with the incorporation of omega 6 PUFA, which has a lower melting point than natural coconut oil.

The present invention also provides a process for the manufacture cholesterol lowering structured lipids from coconut oil.

### DETAILED DESCRIPTION OF THE INVENTION

Accordingly, the present invention provides a process for production of cholesterol lowering structured lipid rich in omega-6-polyunsaturated fatty acid from coconut oil, said process comprising:
(a) hydrolyzing triglycerides of a vegetable oil rich in omega 6 polyunsaturated fatty acid to obtain free fatty acids containing omega 6 polyunsaturated fatty acid,
(b) interesterifying coconut oil with the free fatty acid in a molar ratio of 1:3 at a temperature in the range of 37 to 55°C for a period of 6 to 48 hours in the presence of a solvent and immobilized sn-1-3 lipase to obtain a modified structured lipid, and
(c) separating the modified structured lipid by adsorption chromatography using a solvent selected from hexane and ether to obtain a cholesterol lowering structured lipid rich in omega-6-polyunsaturated fatty acid.

The hydrolysis of triglycerides is done by a known method.

The solvent of step (b) is an hydrocarbon selected from petroleum ether, dioxane, isooctane, n-hexane, toluene etc...

The interesterification is controlled by employing immobilized sn-1-3 lipase for enzymatic acidolysis thereby incorporating the required acyl groups into the specific positions of the triacylglycerols.

In an embodiment of the present invention, purification of reaction products is conducted using adsorption chromatography along with solvents selected from hexane and diethyl ether at a ratio ranging from 85: 5 to 95: 5 to obtain structured lipids.

In another embodiment of the present invention, the vegetable oil rich in omega 6 polyunsaturated fatty acid is a vegetable oil rich in linoleic acid.

In yet another embodiment of the present invention, the vegetable oil rich in linoleic acid is a seed oil except coconut oil, cocoa oil and palm nut oil.

In still another embodiment of the present invention, fatty acids of step (a) are obtained from safflower oil.

Further, in yet another embodiment of the present invention, structured lipids are recovered following scale up in the range of 88-92% having cholesterol- lowering capacity in the range of 10-36%.

Another embodiment of the present invention, wherein the interesterification is carried out using lipase enzyme at 5-10% (w/w) of the substrates.

Yet another embodiment of the present invention, wherein *Rhizomucor meihei* an immobilized lipase is used.

Still another embodiment of the present invention, wherein an immobilized lipase *Rhizomucor meihei* that can be utilized up to 25 cycles without loss of activity, is used, thus ensuring economic viability.

Further another embodiment of the present invention, wherein the natural source of triglycerides is from coconut oil and fatty acid is derived from safflower oil for acidolysis reaction.

The present invention also provides a cholesterol lowering structured lipid rich in omega-6-polyunsaturated fatty acid, obtainable by the process as described above.

An embodiment of the present invention, provides unique structured lipids rich in MCFA (Medium Chain Fatty Acid) and n-6 PUFA (Polyunsaturated fatty acid), which is nutritionally beneficial in being hypocholesterolemic and hypotriglyceridemic.

Another embodiment of the present invention, provides structured lipids comprising lauric acid that produces quick energy for critically ill patients.

Yet another embodiment of the present invention, wherein the structured lipids comprise n-6 PUFA to modulate eicosanoid production in immune compromised patients.

Still another embodiment of the present invention, provides structured lipids that possess a very low melting point 12-15 C and remains as a liquid without phase separation.

Yet another embodiment of the present invention, provides structured lipids comprising a cod liver oil fatty acids and triaglycerols of coconut oil for optimal nutrition.

Still another embodiment of the present invention, comprises structured lipids having n-6 PUFA levels from 1.8 % in the unmodified coconut oil to 45.5% in the structured lipid.

Yet another embodiment of the present invention, wherein the structured lipids comprise purity of triglycerides up to 96mg.

In still another embodiment of the present invention, wherein structured lipids are recovered following scale up in the range of 88-92% having cholesterol lowering capacity in the range of 10- 36%.

### BRIEF DESCRIPTION OF THE ACCOMPANIED DIAGRAMS

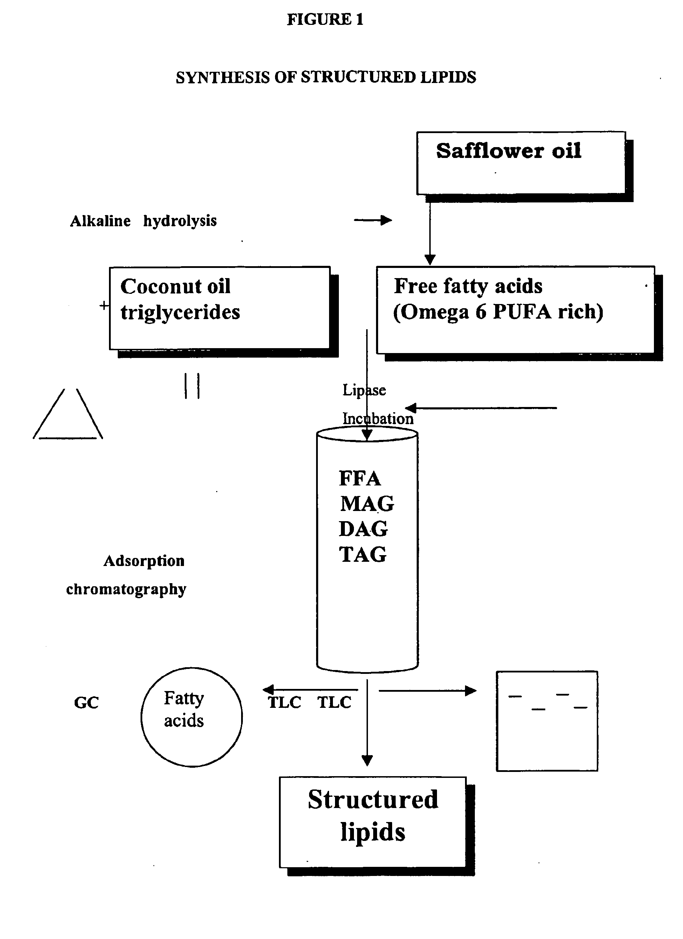

**Fig 1** is a pictorial representation of the steps involved in the synthesis of structured lipids.

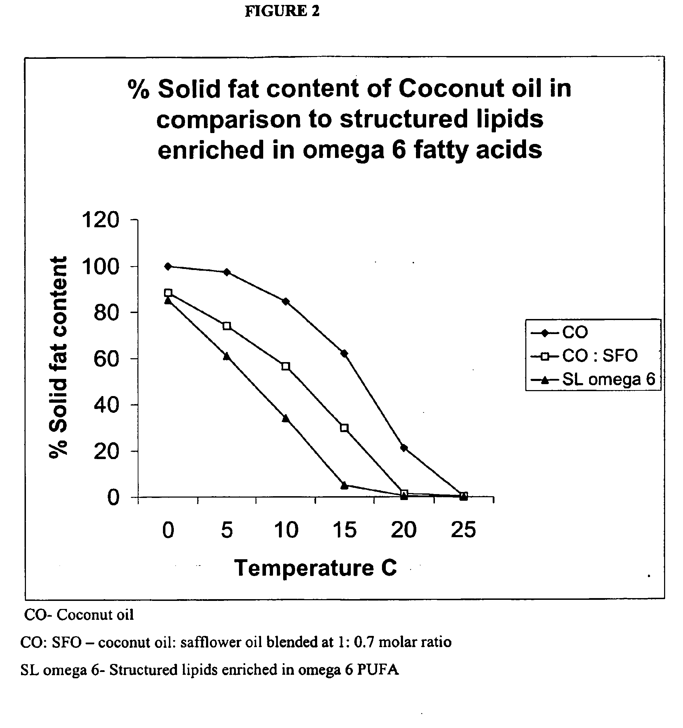

**Fig 2** is a Graphical Diagram depicting the lower melting point of the Structured lipids enriched in Omega 6 PUFA.

The following examples are given by way of illustration of the present invention and therefore should not be construed to limit the scope of the present invention.

### Example 1

Reaction conditions were optimized for the production of structured lipids from coconut oil with safflower oil free fatty acids employing a statistical design (Response surface methodology). Under optimized conditions as predicted by the model for maximum incorporation, 100mg of coconut oil and 132 mg of free fatty acids from safflower oil were taken and 0.5 ml of hexane was added (the volume was maintained through out the reaction). The reaction mixture was taken in a 25 ml conical flask. 11.5 mg of immobilized lipase from *Rhizomucor meihei* was used and the incubation was carried out in an orbitally shaking waterbath at 160 rpm at 39°C for 48.5 hours. The modified triglycerides (structured lipids) was separated by preparative thin layer chromatography with petroleum ether: ethyl ether: acetic acid (80:20:1 v/v/v) as the developing solvent. The analysis of fatty acids by gas chromatography showed a maximum increase in omega 6 PUFA levels from 1.8% in the unmodified coconut oil to 45.5% in the structured lipids. The recovery of structured lipids was 96mg.

### Fatty Acid Composition (mol%) of Unmodified Coconut Oil and Free Fatty Acids Obtained from Safflower Oil and Structured Lipid.

| Fatty acids | **Coconut Oil** | **Safflower Oil Free Fatty Acids** | **Structured Lipid** |
|---|---|---|---|
| Caprylic(8:0) | 2 | - | - |
| Capric(10:0) | 3 | - | - |
| Lauric(12:0) | 48 | - | 17 |
| Myristic(14:0) | 24 | 1 | 11 |
| Palmitic(16:0) | 9 | 8 | 9 |
| Stearic(18:0) | 3 | 3 | 2 |
| Oleic (18:1) | 9 | 20 | 15 |
| Linoleic (18:2) (**omega 6**) | 2 | 68 | 46 |

### Example 2

Enzymatic acidolysis reactions were scaled up in lab scale batch reactions of 100g triglycerides and 132g of free fatty acids from safflower oil taken in a 2 liter Erlenmeyer flask that served as the bioreactor. 11.5g of immobilized lipase was used and the reaction was carried out in 500 ml of hexane. Incubation was carried out for 48.5 hours at 39°C in an orbitally shaking waterbath.

Triglycerides were separated from the reaction mixture by column chromatography. A mixture of 20g each of alumina and silica gel (100-200 mesh ize) were activated at 200°C for 2 hrs and cooled in a desiccator. Slurry of this was made in hexane and packed in 4cm x 35cm glass columns.

30g of the sample from the reaction mixture was loaded on the column and eluted with 350 ml of hexane: diethyl ether (95:5 v/v). The fractions containing triglycerides were pooled and solvent was removed in a vacuum rotary evaporator. The purity of triglycerides was checked by thin layer chromatography. Recovery of the scaled up product was 90g for structured lipids rich in omega 6.

The reactions when carried out at optimized conditions showed 46% incorporation of omega 6 PUFA into coconut oil.

### Example 3

The structured lipids were fed to rats in the diet as the sole source of fat at 10% levels for a period of 60 days. The serum and liver cholesterol was decreased by 10 and 36% respectively and the triglyceride level reduced by 17 and 16% respectively in the serum and liver.

### Lipid Profile of Rats fed Coconut oil, Blends and Structured lipid

| **Dietary Groups** | **Total Cholesterol** | | **Triglycerides** | |
|---|---|---|---|---|
| | **Serum (mg/dl)** | **Liver (mg/g)** | **Serum (mg/dl)** | **Liver (mg/g)** |
| **Coconut oil** | 74.54 ± 1.3 | 7.21 ± 0.2 | 169.90 ± 6.9 | 12.8 ± 2.2 |
| **Coconut oil: Safflower oil (1:0.7) Blend** | 74.76 ± 0.7 | 7.80 ± 1.0 | 169.5 ± 6.7 | 9.07 ± 0.9 |
| **Structured lipid (omega 6 PUFA rich)** | 66.84 ± 1.4 | 4.62 ± 0.3 | 140 ± 4.8 | 6.93 ± 0.7 |

### Example 4

The structured lipids were subjected to DSC studies wherein their peak melting temperature and solid fat content was determined. The peak melting temperature of structured lipids enriched in omega 6 fatty acids was 12.7°C while that of coconut oil was 20.8°C.

### Advantages:

(1) The present invention uses fatty acids from natural sources to create novel structured lipids with potential impacts on nutrition and health
(2) The process could be employed for the modification of coconut oil, which is deficient in EFA by effectively incorporating omega 6 fatty acids.
(3) The production could be scaled up without loosing the efficacy of the process.
(4) The use of natural sources of triglycerides and fatty acids for the acidolysis reaction and the use of immobilized lipase which could be reused up to 25 cycles without loss of activity ensures that the process is economically viable.
(5) Nutritionally the structured lipid proved to be more advantageous than oil blends with similar fatty acid composition or unmodified coconut oil in being hypocholesterolemic and hypotriglyceridemic.
(6) The structured lipid, rich in MCFA and omega 6 PUFA, will provide a lipid source, primarily for use in a controlled diet for critically ill patients, which has lauric acid to provide quick energy and the changes in eicosanoid synthesis seen with omega 6 PUFA feeding will improve immunocompetence and a reduced inflammatory response to injury. Patients in need of elemental diets will benefit from having their immunocompetence improved.
(7) The structured lipid obtained as a result of enzymatic acidolysis reactions displayed an improved melting behavior compared to coconut oil or the oil blends. The structured lipid has a lower melting temperature than unmodified coconut oil thus maintaining them in the liquid state even at low temperatures.. Hence the structured lipid at a temperature of 12 - 15°C remains a liquid without phase separation.

## Claims

1. A process for production of cholesterol lowering structured lipid rich in omega-6-polyunsaturated fatty acid from coconut oil, said process comprising:
(a) hydrolyzing triglycerides of a vegetable oil rich in omega 6 polyunsaturated fatty acid to obtain free fatty acids containing omega 6 polyunsaturated fatty acid,
(b) interesterifying coconut oil with the free fatty acid in a molar ratio of 1:3 at a temperature in the range of 37 to 55°C for a period of 6 to 48. hours in the presence of a solvent and immobilized sn-1-3 lipase to obtain a modified structured lipid, and
(c) separating the modified structured lipid by adsorption chromatography using a solvent selected from hexane and ether to obtain a cholesterol lowering structured lipid rich in omega-6-polyunsaturated fatty acid.

2. A process as claimed in claim 1, wherein the vegetable oil rich in omega 6 polyunsaturated fatty acid is a vegetable oil rich in linoleic acid.

3. A process as claimed in claim 2, wherein the vegetable oil rich in linoleic acid is a seed oil except coconut oil, cocoa oil and palm nut oil.

4. A process as claimed in any one of claims 1 to 3, wherein fatty acids of step (a) are obtained from safflower oil.

5. A process as claimed in any one of claims 1-4, wherein the ether of step (c) is petroleum ether or diethyl ether.

6. A process as claimed in any one of claims 1-5, wherein the solvent of step (b) is selected from petroleum ether, dioxane, isooctane, n-hexane, toluene.

7. A process as claimed in any one of claims 1-6, wherein the ratio of ether:hexane of step (c) is 85:5 to 95:5.

8. A process as claimed in any one of claims 1-7, wherein the amount of lipase enzyme is 5-10% w/w of the substrate.

9. A process as claimed in any one of claims 1-8, wherein the lipase is obtained from *Rhizomucor meihei.*

10. A process as claimed in any one of claims 1-9, wherein the lipase is obtained from *Rhizomucor meihei* and is used up to 25 cycles without loss of activity.

11. A process as claimed in any one of claims 1-10, wherein the recovery of structured lipids from the reaction mixture after interesterification reaction is in the range of 88-92%.

12. A cholesterol lowering structured lipid rich in omega-6-polyinsaturated fatty acid, obtainable by the process as claimed in any one of claims 1-11.

13. The structured lipid as claimed in claim 12, containing up to 46 mol % of omega-6-polyunsaturated fatty acids and rich in medium chain fatty acids.

14. A structured lipid as claimed in claim 12, comprising:
| | | |
|---|---|---|
| Lauric acid | : | 17% |
| Myristic acid | : | 11% |
| Palmitic acid | : | 9 % |
| Stearic acid | : | 2 % |
| Oleic acid | : | 15 % |
| Linoleic acid | : | 46 % |

15. Use of a cholesterol lowering structured lipid as claimed in any one of claims 12 to 14, for manufacturing a composition for being clinically administrated to patients in parenteral nutrition.

16. Use of the cholesterol lowering structured lipid as defined in any one of claims 12-14, for manufacturing a composition comprising lauric acid for providing quick energy for critically ill patients.

17. Use of the cholesterol lowering structured lipid as defined in any one of claims 12-14, for manufacturing a composition comprising omega-6-polyunsaturated fatty acids for modulating the eicosanoid production in immune compromised patients.

18. Use of the cholesterol lowering structured lipid as defined in any one of claims 12-14, for manufacturing a composition for nutritional benefits due to hypocholesterolemic and hypotriglyceridemic.

19. Use of the cholesterol lowering structured lipid as defined in any one of claims 12-14, for manufacturing a composition for controlled diet for critically ill patients.

## Patentansprüche

1. Verfahren zur Herstellung eines cholesterinsenkenden strukturierten Lipids, das reich an Omega-6-polyungesättigter Fettsäure ist, aus Kokosöl, wobei das Verfahren umfaßt:
(a) Hydrolysieren von Trigylceriden aus einem Pflanzenöl, das reich an Omega-6-polyungesättigter Fettsäure ist, um freie Fettsäuren zu erhalten, die Omega-6-polyungesättigte Fettsäure enthalten,
(b) Untereinander Verestern von Kokosöl mit der freien Fettsäure in einem Molverhältnis von 1:3 bei einer Temperatur im Bereich von 37 bis 55° C für einen Zeitraum von 6 bis 48 Stunden in Gegenwart eines Lösungsmittels und immobilisierter sn-1,3-Lipasen, um ein modifiziertes strukturiertes Lipid zu erhalten, und
(c) Trennen des modifizierten strukturierten Lipids durch Adsorptionschromatographie unter Verwendung eines Lösungsmittels, das gewählt ist aus Hexan und Ether, um ein cholesterinsenkendes strukturiertes Lipid zu erhalten, das reich an Omega-6-polyungesättigter Fettsäure ist.

2. Verfahren nach Anspruch 1, wobei das Pflanzenöl, das reich an Omega-6-polyungesättigter Fettsäure ist, ein Pflanzenöl ist, das reich an Linolsäure ist.

3. Verfahren nach Anspruch 2, wobei das Pflanzenöl, das reich an Linolsäure ist, ein Samenöl außer Kokosöl, Kakaoöl und Palmnußöl ist.

4. Verfahren nach einem der Ansprüche 1 bis 3, wobei Fettsäuren von Schritt (a) erhalten werden aus Distelöl.

5. Verfahren nach einem der Ansprüche 1 bis 4, wobei der Ether von Schritt (c) Petrolether oder Diethylether ist.

6. Verfahren nach einem der Ansprüche 1 bis 5, wobei das Lösungsmittel von Schritt (b) gewählt ist aus Petrolether, Dioxan, Isooctan, n-Hexan, Toluol.

7. Verfahren nach einem der Ansprüche 1 bis 6, wobei das Verhältnis Ether:Hexan von Schritt (c) 85:5 bis 95:5 ist.

8. Verfahren nach einem der Ansprüche 1 bis 7, wobei die Menge an Lipaseenzym 5 - 10% Gewicht/Gewicht des Substrats ist.

9. Verfahren nach einem der Ansprüche 1 bis 8, wobei die Lipase erhalten ist aus *Rhizomucor meihei.*

10. Verfahren nach einem der Ansprüche 1 bis 9, wobei die Lipase erhalten ist aus *Rhizomucor meihei* und bis zu 25 Zyklen ohne Aktivitätsverlust verwendet wird.

11. Verfahren nach einem der Ansprüche 1 bis 10, wobei die Ausbeute an strukturierten Lipiden aus dem Reaktionsgemisch nach Veresterungsreaktion untereinander im Bereich von 88 - 92% liegt.

12. Cholesterinsenkendes strukturiertes Lipid, das reich an Omega-6-polyungesättigter Fettsäure ist, erhältlich durch das Verfahren nach einem der Ansprüche 1 bis 11.

13. Strukturiertes Lipid nach Anspruch 12, weiches bis zu 46 mol-°/a Omega-6-polyungesättigte Fettsäuren enthält und reich an mittelkettigen Fettsäuren ist.

14. Strukturiertes Lipid nach Anspruch 12, umfassend:
| | | |
|---|---|---|
| Laurinsäure | : | 17% |
| Myristinsäure | : | 11% |
| Palmitinsäure | : | 9% |
| Stearinsäure | : | 2% |
| Ölsäure | : | 15% |
| Linolsäure | : | 46% |

15. Verwendung eines cholesterinsenkenden strukturierten Lipids nach der Ansprüche 12 bis 14 zur Herstellung einer Zusammensetzung, um klinisch verabreicht zu werden an Patienten bei künstlicher Ernährung.

16. Verwendung des cholesterinsenkenden strukturierten Lipids nach einem der Ansprüche 12 bis 14 zur Herstellung einer Zusammensetzung, welche Laurinsäure umfaßt, zur schnellen Bereitstellung von Energie für schwerkranke Patienten.

17. Verwendung des cholesterinsenkenden strukturiertem Lipids nach einem der Absprüche 12 bis 14 zur Herstellung einer Zusammensetzung, welche Omega-6-polyungesättigte Fettsäuren umfaßt, zur Modulierung der Eicosanoidproduktion in immunbeeinträchtigten Patienten.

18. Verwendung des cholesterinsenkenden strukturierten Lipids nach einem des Ansprüche 12 bis 14, zur Herstellung einer Zusammensetzung für Ernährungsvorteite aufgrund von Hypocholesterolämie und Hypertriglyceridämie.

19. Verwendung des cholesterinsenkenden strukturierten Lipids nach einem der Ansprüche 12 bis 14 zur Herstellung einer Zusammensetzung zur kontrollierten Diät für schwerkranke Patienten.

## Revendications

1. Procédé pour la production de lipide structuré abaissant le cholestérol riche en acide gras oméga-6-polyinsaturé provenant de l'huile de copra, ledit procédé comprenant:
(a) l'hydrolyse de triglycérides d'une huile végétale riche en acide gras oméga 6 polyinsaturé pour obtenir des acides gras libres contenant un acide gras oméga 6 polyinsaturé,
(b) l'interestérification d'huile de copra avec l'acide gras libre dans un rapport molaire de 1:3 à une température dans la plage de 37 à 55°C pendant une durée de 6 à 48 heures en présence d'un solvant et de sn-1-3 lipase immobilisée pour obtenir un lipide structuré modifié, et
(c) la séparation du lipide structuré modifié par chromatographie d'adsorption au moyen d'un solvant choisi parmi l'hexane et un éther pour obtenir un lipide structuré abaissant le cholestérol riche en acide gras oméga-6-polyinsaturé.

2. Procédé selon la revendication 1, où l'huile végétale riche en acide gras oméga 6 polyinsaturé est une huile végétale riche en acide linoléique.

3. Procédé selon la revendication 2, où l'huile végétale riche en acide linoléique est une huile de graines sauf l'huile de copra, l'huile de cacao et l'huile de palmiste.

4. Procédé selon l'une quelconque des revendications 1 à 3, où les acides gras de l'étape (a) sont obtenus à partir d'huile de carthame.

5. Procédé selon l'une quelconque des revendications 1-4, où l'éther de l'étape (c) est l'éther de pétrole ou le diéthyléther.

6. Procédé selon l'une quelconque des revendications 1-5, où le solvant de l'étape (b) est choisi parmi l'éther de pétrole, le dioxane, l'isooctane, le n-hexane, le toluène.

7. Procédé selon l'une quelconque des revendications 1-6, où le rapport éther:hexane de l'étape (c) est 85:5 à 95:5.

8. Procédé selon l'une quelconque des revendications 1-7, où la quantité d'enzyme lipase est 5-10 % p/p du substrat.

9. Procédé selon l'une quelconque des revendications 1-8, où la lipase est obtenue à partir de *Rhizomucor meihei.*

10. Procédé selon l'une quelconque des revendications 1-9, où la lipase est obtenue à partir de *Rhizomucor meihei* et est utilisée jusqu'à 25 cycles sans perte d'activité.

11. Procédé selon l'une quelconque des revendications 1-10, où la récupération de lipides structurés à partir du mélange réactionnel après la réaction d'interestérification est dans la plage de 88-92 %.

12. Lipide structuré abaissant le cholestérol riche en acide gras oméga-6-polyinsaturé pouvant être obtenu par le procédé selon l'une quelconque des revendications 1-11.

13. Lipide structuré selon la revendication 12, contenant jusqu'à 46 mol % d'acides gras oméga-6-polyinsaturés et riche en acides gras à chaîne moyenne.

14. Lipide structuré selon la revendication 12, comprenant :
| | | |
|---|---|---|
| Acide laurique | : | 17% |
| Acide myristique | : | 11 % |
| Acide palmitique | : | 9 % |
| Acide stéarique | : | 2 % |
| Acide oléique | : | 15 % |
| Acide linoléique | : | 46 % |

15. Utilisation d'un lipide structuré abaissant le cholestérol selon l'une quelconque des revendications 12 à 14, pour fabriquer une composition destinée à être administrée cliniquement à des patients en nutrition parentérale.

16. Utilisation du lipide structuré abaissant le cholestérol selon l'une quelconque des revendications 12-14 pour fabriquer une composition comprenant de l'acide laurique pour fournir de l'énergie rapide pour des patients malades de manière critique.

17. Utilisation du lipide structuré abaissant le cholestérol selon l'une quelconque des revendications 12-14 pour fabriquer une composition comprenant des acides gras oméga-6-polyinsatu rés pour moduler la production d'eicosanoïdes chez des patients immunocompromis.

18. Utilisation du lipide structuré abaissant le cholestérol selon l'une quelconque des revendications 12-14 pour fabriquer une composition pour des bénéfices nutritionnels car hypocholestérolémique et hypotriglycéridémique.

19. Utilisation du lipide structuré abaissant le cholestérol selon l'une quelconque des revendications 12-14 pour fabriquer une composition pour un régime contrôlé pour des patients malades de manière critique.
